# EUROPEAN PATENT APPLICATION

(11) **EP 4 656 737 A1**
(43) Date of publication of application: **03.12.2025**
(21) Application number: 23918721.4
(22) Date of filing: 12.05.2023
(51) Int. Cl.: C12Q 1/6883, G01N 33/68, A61K 48/00, A61P 1/16

(54) **BIOMARKER COMPOSITION FOR LIVER DISEASE DIAGNOSIS AND PHARMACEUTICAL COMPOSITION FOR TREATMENT CONTAINING LRG1 PROTEIN OR CODING GENE THEREFOR**

(30) Priority: 27.01.2023 KR 20230010999
(71) Applicant: Kyungpook National University Industry-Academic Cooperation Foundation, Daegu 41566 (KR)
(72) Inventor: LEE, You Mie, Chilgok-gun Gyeongsangbuk-do 39855 (KR)
(74) Representative: Mewburn Ellis LLP
(86) International application number: PCT/KR2023/006504
(87) International publication number: WO 2024/158086

(57) **Abstract**

The present invention relates to a biomarker composition for the diagnosis of liver diseases. It has been confirmed that Lrgl increases in cases of liver fibrosis, indicating that Lrgl can be utilized for the diagnosis of liver diseases, including liver fibrosis. Furthermore, the potential of Lrgl as a therapeutic target for liver diseases has been identified, allowing its use in the prevention or treatment of liver diseases.

## Description

### Technical Field

The present disclosure provides a biomarker composition for diagnosing a liver disease and a pharmaceutical composition for treatment, including an Lrgl protein or a gene encoding the same.

### Background Art

A liver disease is caused by various factors such as alcohol, various drugs, toxic chemicals, hepatitis B and C viruses, cholestasis, and autoimmune diseases, and usually progresses from fatty liver to hepatitis, liver fibrosis, and cirrhosis. A fatty liver disease is not a pathological state itself, but rather a reversible symptom that naturally recovers when the cause is eliminated. However, if a state in which excessive fat accumulates in the liver tissue is maintained, steatohepatitis occurs to result in repeated hepatocyte necrosis and regeneration, during which liver fibrosis progresses due to the increase and accumulation of fibrous extracellular matrix (ECM).

Liver fibrosis remains one of the major diseases in the world's population. Fibrosis is a result of chronic damage to the liver with the accumulation of extracellular matrix (ECM) proteins such as collagen, fibronectin, and laminin, and characteristics of most types of chronic disease is also caused by repeated liver damage with persistent inflammation, scar tissue formation, changes in tissue conformation, and organ failure. Hepatic stellate cells (HSCs) are notable as cells that play a key role in liver fibrosis, since they are the main source of fibrillar and nonfibrillar matrix proteins and are a central process of fibrosis. Quiescent HSCs produce less ECM proteins than activated HSCs, but when repetitive damage changes quiescent HSCs into an activated state, they proliferate and transform into a myofibroblast-like phenotype, a process that is termed activation.

Activated HSC function causes excessive accumulation of ECM and destroys the normal liver architecture, thereby causing pathophysiological damage to the liver. Overexpression of ECM proteins ultimately leads to liver failure, fibrosis, cirrhosis, or cancer by HSC activation. Abnormal state of ECM proteins, including accumulation and overexpression in liver tissues, leads to excessive hepatic matrix in the late-stage liver disease or disorder. This condition is the most critical stage during a progressive liver disease. Therefore, prevention and suppression of liver fibrosis are very important for alleviating the chronic liver disease. Nevertheless, to date, no FDA-approved treatment for liver fibrosis has been developed, and the specific mechanism by which a liver cancer develops by liver fibrosis has not been elucidated.

Therefore, in order to solve the above problem, it is necessary to develop methods and therapeutic agents for diagnosing a liver disease including liver fibrosis.

### Disclosure of the Invention

### Technical Goals

An object of the present disclosure is to provide a biomarker composition for diagnosing a liver disease, including a leucine-rich alpha-2-glycoprotein 1 (Lrgl) protein or a gene encoding the same.

Another object of the present disclosure is to provide a composition for diagnosing a liver disease, containing an agent capable of measuring an expression or activity level of an Lrgl protein, or an expression level of a gene encoding the protein as an active ingredient.

Another object of the present disclosure is to provide a kit for diagnosing a liver disease including the composition.

Another object of the present disclosure to provide a method of providing information necessary for diagnosing a liver disease, including: (1) measuring an mRNA expression level of an Lrgl gene or an expression level of an Lrgl protein from a sample isolated from a patient with a liver disease; (2) comparing the mRNA expression level of the Lrgl gene or the expression level of the Lrgl protein with that of a control sample; and (3) determining as a liver disease if the mRNA expression level of the Lrgl gene or the expression level of the Lrgl protein is higher than that of the control sample.

Another object of the present disclosure is to provide a pharmaceutical composition for preventing or treating a liver disease, including an Lrgl expression or activity inhibitor as an active ingredient.

Another object of the present disclosure is to provide a method of screening an agent for treating liver disease, including: (1) contacting a test substance with liver disease cells; (2) measuring an expression or activity level of an Lrg1 protein in the liver disease cells that have come into contact with the test substance; and (3) selecting a test substance having a reduced expression or activity level of the Lrgl protein compared to a control sample.

### Technical Solutions

To achieve the above objects, the present disclosure provides a biomarker composition for diagnosing a liver disease, including a leucine-rich alpha-2-glycoprotein 1 (Lrgl) protein or a gene encoding the same.

In addition, the present disclosure provides a composition for diagnosing a liver disease, containing an agent capable of measuring an expression or activity level of an Lrgl protein, or an expression level of a gene encoding the protein as an active ingredient.

In addition, the present disclosure provides a kit for diagnosing a liver disease, including the composition.

In addition, the present disclosure provides a method of providing information necessary for diagnosing a liver disease, including: (1) measuring an mRNA expression level of an Lrgl gene or an expression level of an Lrgl protein from a sample isolated from a patient with a liver disease; (2) comparing the mRNA expression level of the Lrgl gene or the expression level of the Lrgl protein with that of a control sample; and (3) determining as a liver disease if the mRNA expression level of the Lrgl gene or the expression level of the Lrgl protein is higher than that of the control sample.

In addition, the present disclosure provides a pharmaceutical composition for preventing or treating a liver disease, including an Lrgl expression or activity inhibitor as an active ingredient.

In addition, the present disclosure provides a method of screening an agent for treating liver disease, including: (1) contacting a test substance with liver disease cells; (2) measuring an expression or activity level of an Lrgl protein in the liver disease cells that have come into contact with the test substance; and (3) selecting a test substance having a reduced expression or activity level of the Lrgl protein compared to a control sample.

### Advantageous Effects

The present disclosure relates to a biomarker composition for diagnosing a liver disease, wherein, in the case of liver fibrosis, by detecting an increase in Lrgl, Lrgl may be utilized in the diagnosis of a liver disease including liver fibrosis, and furthermore, by confirming a potential of Lrgl as a therapeutic target for a liver disease, it may be utilized for prevention or treatment of a liver disease.

### Brief Description of Drawings

FIG. 1 shows results of identifying that inflammation increases in Runx3 deficient cells.
FIG. 2 shows a method of preparing a Runx3 deficient mouse.
FIG. 3 shows results of identifying hepatic vascular endothelial cell dysfunction in Runx3 deficient mice.
FIG. 4 shows results of identifying symptoms of liver fibrosis in Runx3 deficient mice.
FIG. 5 shows results of identifying that Lrgl increases in Runx3 deficient cells.
FIG. 6 shows results of identifying that Lrgl is secreted through an IL-6/JAK/STAT3 pathway in Runx3 deficient cells.
FIG. 7 shows results of identifying that Lrgl increases α-SMA and collagen I through SMAD2 and SMAD3 signals in HSCs.
FIG. 8 shows results of identifying that HSC activation of Lrgl is induced by TGF-βR signal.
FIG. 9 shows a result of identifying that Lrgl increases α-SMA and collagen I independently of TGF-β.
FIG. 10 shows results of identifying that Lrgl increases in liver fibrosis and further increases in a Runx3^{ΔEC} mouse model.

### Best Mode for Carrying Out the Invention

Hereinafter, the present disclosure will be described in more detail.

The present disclosure provides a biomarker composition for diagnosing a liver disease, including a leucine-rich alpha-2-glycoprotein 1 (Lrgl) protein or a gene encoding the same.

The liver disease may be any one selected from the group consisting of liver fibrosis, liver cancer, hepatitis, hepatotoxicity, alcoholic fatty liver disease, and non-alcoholic fatty liver disease, but is not limited thereto.

The term "diagnosis" as used herein includes determining the susceptibility of an individual to a particular disease or disorder, determining whether an individual currently has a particular disease or disorder, determining the prognosis of an individual with a particular disease or disorder, or therametrics (e.g., monitoring a status of an individual to provide information on an efficacy of a treatment).

The term "biomarker" as used herein refers to a substance capable of diagnosing tissues or cells of a subject with a liver disease developed, by distinguishing them from tissues or cells of a normal control group, including organic biomolecules such as proteins or nucleic acids, lipids, glycolipids, and glycoproteins that show an increase or decrease pattern in tissues or cells of a subject with the disease developed compared to a normal control group.

In addition, the present disclosure provides a composition for diagnosing a liver disease, containing an agent capable of measuring an expression or activity level of an Lrgl protein, or an expression level of a gene encoding the protein as an active ingredient.

The agent capable of measuring the expression level of the Lrgl may be, but is not limited to, a primer or probe that specifically binds to the Lrgl gene, or an antibody, peptide, aptamer, or compound that specifically binds to the Lrgl protein.

The term "primer" as used herein refers to an oligonucleotide synthesized for the purpose of diagnosis, DNA sequencing, etc., as a short genetic sequence that serves as an initiation site of DNA synthesis. The primers may be utilized conventionally by synthesizing in a length of 15 to 30 base pairs but vary depending on the intended use, and it may be modified by methylation and capping via known methods.

The term "probe" as used herein refers to a nucleic acid capable of specifically binding to mRNA in a few bases to several hundred bases in length, prepared through an enzymatic chemical separation/purification or synthetic process. The presence of mRNA may be detected by labeling radioactive isotopes or enzymes, and it may be designed and modified to be used via known methods.

The term "antibody" as used herein as a term known in the art refers to a specific immunoglobulin directed against an antigenic site. The antibody as used herein refers to an antibody that specifically binds to Lrgl of the present disclosure, and the antibody may be prepared according to a conventional method known in the art. The form of the antibodies includes polyclonal antibodies or monoclonal antibodies and encompasses all immunoglobulin antibodies. The antibody refers to a complete form having two full-length light chains and two full-length heavy chains. Additionally, the antibodies include special antibodies such as humanized antibodies.

The term "peptide" as used herein has high binding affinity to a target substance without undergoing denaturation even with heat/chemical treatment. Additionally, owing to a small molecular size, it may be attached to other proteins to be used as a fusion protein. Specifically, since it may be used by being attached to a polymer protein chain, it may be used as a diagnostic kit and drug delivery substance.

The term "aptamer" as used herein refers to a type of polynucleotide composed of a special type of single-stranded nucleic acid (DNA, RNA, or modified nucleic acid) that has a stable tertiary structure in itself and exhibits characteristics of being able to bind to a target molecule with high affinity and specificity. As described above, the aptamer may specifically bind to antigenic substances in the same way as antibodies, but it is more stable than proteins with a simple structure and used as a substitute for antibodies since it is composed of polynucleotides that are easily synthesizable.

In addition, the present disclosure provides a kit for diagnosing a liver disease, including the composition.

The "kit" as used herein may include an antibody that specifically binds to a biomarker component, a secondary antibody conjugate to which a label that develops color by reaction with a substrate is conjugated, a chromogenic solution that reacts with the label to develop color, a washing solution, and an enzymatic reaction stop solution, and may be manufactured into a plurality of separate packages or compartments containing reagent components to be used.

In addition, the present disclosure provides a method of providing information necessary for diagnosing a liver disease, including (1) measuring an mRNA expression level of an Lrgl gene or an expression level of an Lrgl protein from a sample isolated from a patient with a liver disease; (2) comparing the mRNA expression level of the Lrgl gene or the expression level of the Lrgl protein with that of a control sample; and (3) determining as a liver disease if the mRNA expression level of the Lrgl gene or the expression level of the Lrgl protein is higher than that of the control sample.

In addition, the present disclosure provides a pharmaceutical composition for preventing or treating a liver disease, including an Lrgl expression or activity inhibitor as an active ingredient.

The Lrgl expression inhibitor may be any one selected from the group consisting of an antisense nucleotide, a small interfering RNA (siRNA), and a short hairpin RNA (shRNA) that complementarily bind to mRNA of an Lrgl gene, but is not limited thereto.

The Lrgl activity inhibitor may be any one selected from the group consisting of small molecule compounds, peptides, peptide mimetics, aptamers, antibodies, and natural products that specifically bind to an Lrgl protein, but is not limited thereto.

In other embodiments of the present disclosure, the pharmaceutical composition may further include one or more additives selected from the group consisting of appropriate carriers, excipients, disintegrators, sweeteners, coating agents, leavening agents, lubricants, glydents, flavoring agents, antioxidants, buffers, bacteriostatic agents, diluents, dispersants, surfactants, binders, and lubricants that are commonly used in the manufacture of pharmaceutical compositions. Specifically, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearate, and mineral oil may be used as the carriers, excipients, and diluents, solid preparations for oral administration may include tablets, pills, acids, granules, and capsules, and these solid preparations may be prepared by mixing, in the composition, at least one or more excipients, for example, starch, calcium carbonate, sucrose or lactose, and gelatin. In addition to simple excipients, lubricants such as magnesium stearate and talc may also be used. Oral liquid preparations may include suspensions, liquids, emulsions, and syrups, and various excipients, such as humectants, sweeteners, fragrances, and preservatives may be included in addition to the simple diluents that are commonly used, such as water and liquid paraffin. Preparations for parenteral administration include sterile aqueous solutions, non-aqueous solvents, suspensions, emulsions, lyophilized agents, and suppositories. For non-aqueous solvents and suspensions, propylene glycol, polyethylene glycol, vegetable oils such as olive oil, and injectable esters such as ethyl oleate may be used. As for base materials of suppositories, witepsol, macrogol, tween 61, cacao butter, laurin fat, and glycerogelatin may be used. According to one embodiment of the present disclosure, the pharmaceutical composition may be administered to a subject in a conventional manner through intravenous, intraarterial, intraperitoneal, intramuscular, intraarterial, intraperitoneal, intrasternal, transdermal, intranasal, inhalation, topical, rectal, oral, intraocular, or intradermal routes. The dosage of the active ingredient according to the present disclosure may vary depending on the condition and body weight of the subject, the type and severity of the disease, the form of the drug, and the route and duration of administration and may be appropriately selected by those skilled in the art, and the daily dose may be 0.01 mg/kg to 200 mg/kg, preferably 0.1 mg/kg to 200 mg/kg, and more preferably 0.1 mg/kg to 100 mg/kg. The administration may be conducted once a day or divided into several doses, but the scope of the present disclosure is not limited thereby.

In addition, the present disclosure provides a method of screening an agent for treating liver disease, including (1) contacting a test substance with liver disease cells; (2) measuring an expression or activity level of an Lrgl protein in the liver disease cells that have come into contact with the test substance; and (3) selecting a test substance having a reduced expression or activity level of the Lrgl protein compared to a control sample.

The Lrgl expression level may be measured by any one or more selected from the group consisting of reverse transcription-polymerase chain reaction (RT-PCR), enzyme-linked immunosorbent assay (ELISA), radioimmunoassay, immunohistochemistry, microarray, western blotting, and flow cytometry (FACS), but is not limited thereto.

Hereinafter, the present disclosure will be described in more detail through examples to help understanding of the present disclosure. However, examples below are merely intended to illustrate the content of present disclosure, and the scope of the present disclosure is not limited to the following examples. Examples of the present disclosure are provided to more completely explain the present disclosure to those skilled in the art.

### Modes for Carrying Out the Invention

### <Experimental Example 1> Identification of changes in cytokines due to Runx3 deficiency in human liver sinusoidal endothelial cells

To identify the changes in cytokines due to Runx3 deficiency in human liver sinusoidal endothelial cells (LSECs), TMNK-1 cells (human immortalized LSECs) were transfected with siRunx3 and control siRNA, and after 36 hours, total RNA was isolated, followed by qRT-PCR.

### <Experimental Example 2> Preparation of Runx3 deficient mouse models

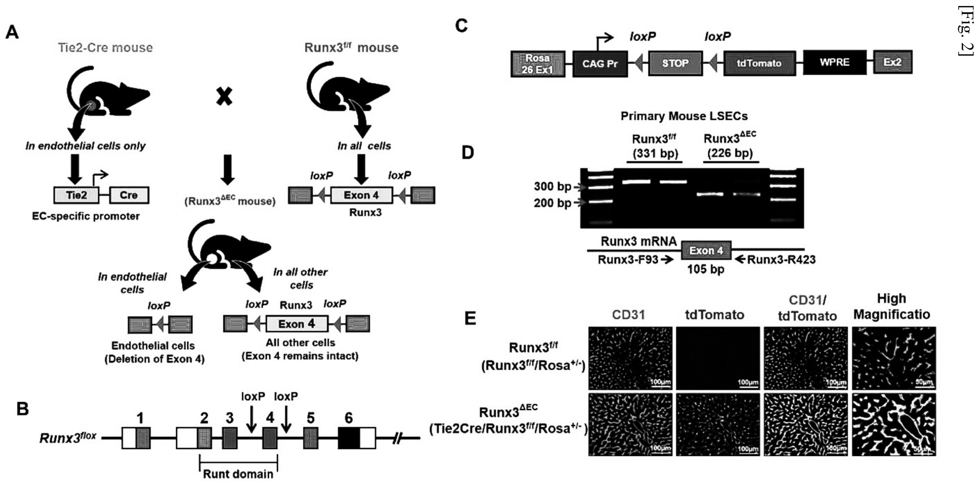

Endothelial cells (hereinafter referred to as ECs)-specific Runx3 deficient mice were prepared using the tissue-specific Cre-loxP system. As shown in FIG. 2, mice (Tie2-Cre mouse) in which Cre recombinase and Tie2 promoter are cloned to specifically activate Cre in the EC where Tie2 is expressed were crossed with Runx3 floxed mice (Runx3^{f/f} mouse) in which a gene created by inserting loxP sites in front and behind exon4 of Runx3 is expressed, to create mice in which Runx3 exon4 is deleted in the EC.

### <Experimental Example 3> Immunohistochemistry

After fixing the mouse liver in formaldehyde, a paraffin block was created, and the paraffin tissue block was cut into 5 µm thick pieces to be placed on slides for use. Paraffin was removed from the paraffin tissue sections, and after going through several processes for hydration, treatment was performed in a citrate solution (pH 6.0) at 95°C for 10 to 20 minutes, and then immunohistochemistry (hereinafter referred to as IHC) was followed. After treatment in 3% H₂O₂ solution for 10 minutes, blocking was carried out with 10% goat serum and left overnight in a primary antibody solution at 4°C. After the appropriate dilution of biotin-labeled secondary antibodies in a solution containing goat serum for 1 hour, a reaction was carried out with VECTASTAIN Elite ABC solution for 30 minutes, followed by staining with treatment of a DAB substrate kit. Frozen tissue section slides were also used. After undergoing blocking of the tissue sections with 3% BSA/PBS solution, the primary antibody was left overnight in a diluted solution (1% BSA. 0.3% triton X-100) at 4°C. After treatment with a secondary fluorescent antibody solution for 1 hour, mounting was performed with a solution containing DAPI solution, followed by observation under a fluorescence microscope or confocal microscope.

### <Experimental Example 4> Western Blot

Liver tissues were mixed with a loading solution and boiled at 95°C for 10 minutes, protein was separated by size using SDS-PAGE electrophoresis, gel was transferred to a 0.45 µm nitrocellulose membrane, and the membrane was blocked with 5% skim milk. After being left overnight at 4°C with the primary antibody solution, the protein bands were identified with ECL substrate after reaction with HRP-conjugated secondary antibody for 1 hour.

### <Experimental Example 5> Measurement of serum ALT and AST

Coagulation was performed in mouse serum for 1 hour followed by centrifugation at 4°C and 1500g for 15 minutes, and measurement was carried out using AST, ALT kit (Bio-Vision).

### <Experimental Example 6> Histological staining

The paraffin tissue slides were left at 68°C for 1 hour, hydrated with various concentrations of ethanol solutions, rinsed with water, and then stained according to standard protocols for H&E staining, Masson's trichrome staining, and Sirius red staining.

### <Experimental Example 7> Single cell RNA-sequencing

Hepatocytes and other cells were separated from each mouse group, 2x10⁷ cells were analyzed per group at a ratio of 10% hepatocytes and 90% other cells, and then GOBP analysis was performed. Additionally, protein expression was detected in the tissues via IHC.

### <Example 1> Identification of cytokine changes due to Runx3 deficiency in human liver sinusoidal endothelial cells

qRT-PCR was performed to identify cytokine changes due to Runx3 deficiency in human liver sinusoidal endothelial cells (LSECs).

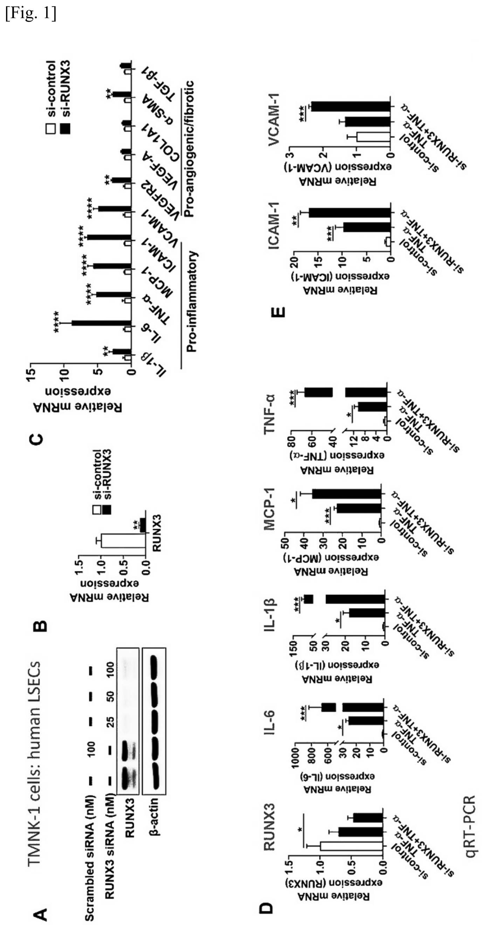

As a result, according to FIG. 1, it was found that the expression levels of IL-1β, IL-6, TNF-α, MCP-1, ICAM-1, and VCAM-1 significantly increased compared to the control group when Runx3 was deficient.

### <Example 2> Identification of symptoms of liver vascular endothelial cell dysfunction and liver fibrosis in Runx3 deficient mice

### (1) Preparation of Runx3 deficient mouse models

Endothelial cells (ECs)-specific Runx3 deficient mice were generated using the tissue-specific Cre-loxP system. As shown in FIG. 2, mice (Tie2-Cre mouse) in which Cre recombinase and Tie2 promoter are cloned to specifically activate Cre in the EC where Tie2 is expressed were crossed with Runx3 floxed mice (Runx3^{f/f} mouse) in which a gene created by inserting loxP sites in front and behind exon4 of Runx3 is expressed, to prepare mice in which Runx3 exon4 is deleted in the EC.

### (2) In case of young Runx3^{ΔEC} mouse model

Genes were identified by immunohistochemistry (IHC analysis) in the young Runx3^{ΔEC} mouse model for proteins that change in response to Runx3 deficiency.

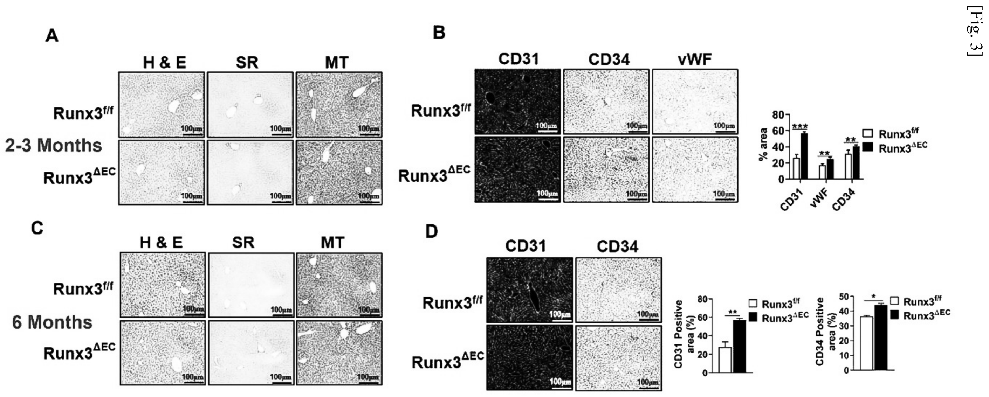

As a result, according to FIG. 3, in the case of the young Runx3^{ΔEC} mouse model that is 2 to 3 months old, it was found that the expression levels of CD34 and vWF increased compared to Runx3^{f/f}, and in the case of the 6-month young Runx3^{ΔEC} mouse model, it was found that the expression levels of CD31 and CD34 increased compared to Runx3^{f/f}.

### (3) In the case of the aged Runx3^{ΔEC} mouse model

Genes that change in response to Runx3 deficiency were identified in an aged Runx3^{ΔEC} mouse model.

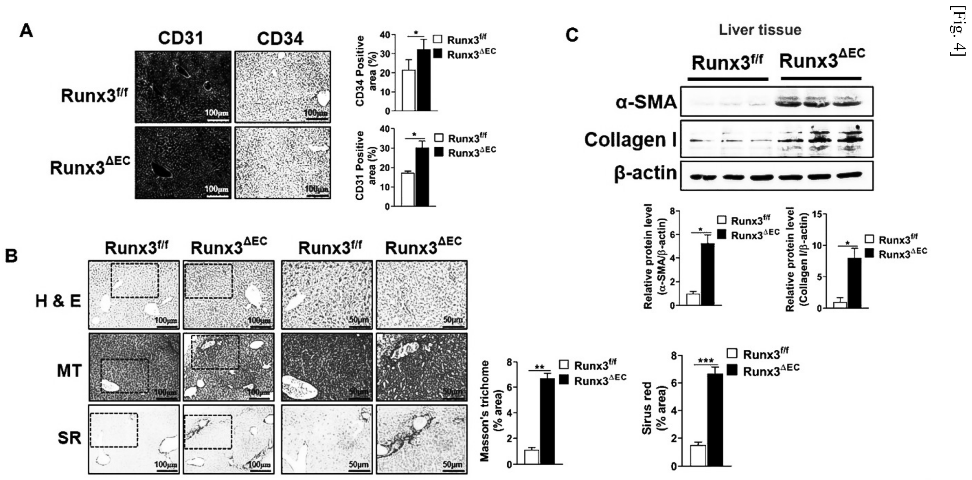

As a result, according to FIG. 4, in the case of the 1-year-old Runx3^{ΔEC} mouse model, it was noticed that the expression levels of CD31 and CD34 increased compared to Runx3^{f/f}, and the expression levels of α-SMA and collagen I in the liver tissue significantly increased, indicating that liver fibrosis symptoms had occurred.

### <Example 3> Identification of an Runx3 deficiency-induced increase in the Lrg1 expression level and secretion pathway

Single cell RNA-sequencing and qRT-PCR were performed to find out which genes' expression changes when Runx3 is deleted in LSEC cells.

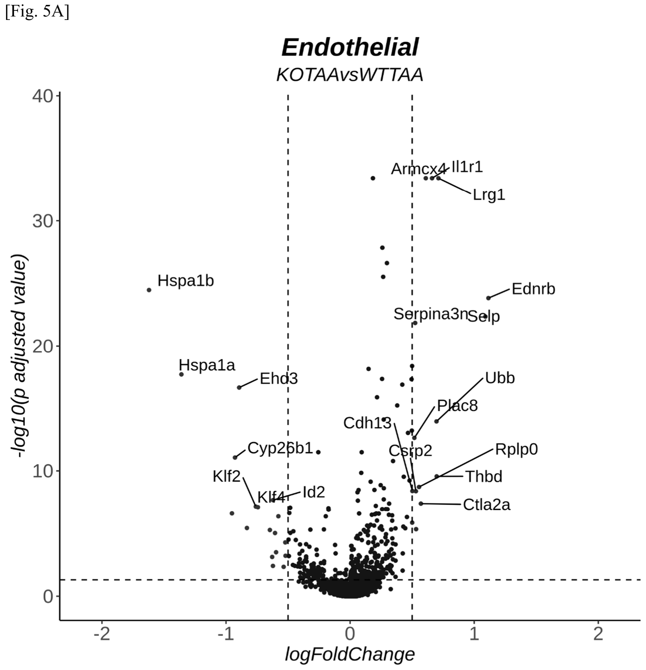

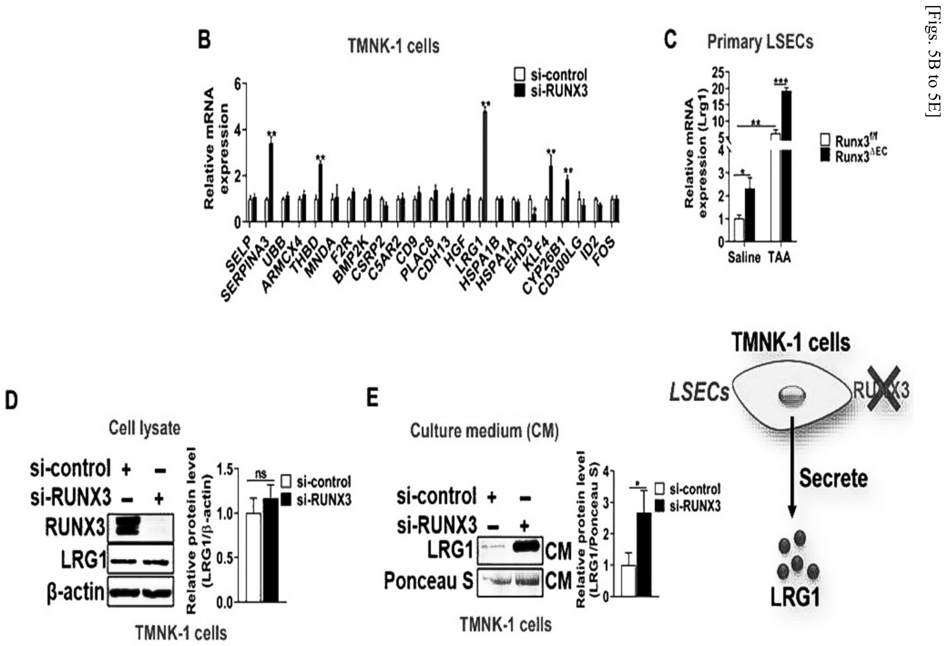

As a result, according to FIG. 5, it was found that the expression level of Lrgl significantly increased compared to the case where Runx3 is not deficient, thereby revealing the possibility of Lrgl as a therapeutic target for liver diseases such as liver fibrosis.

Additionally, Western blot was performed with cell lysates and conditioned media to find a pathway by which Lrgl is secreted in LSEC cells.

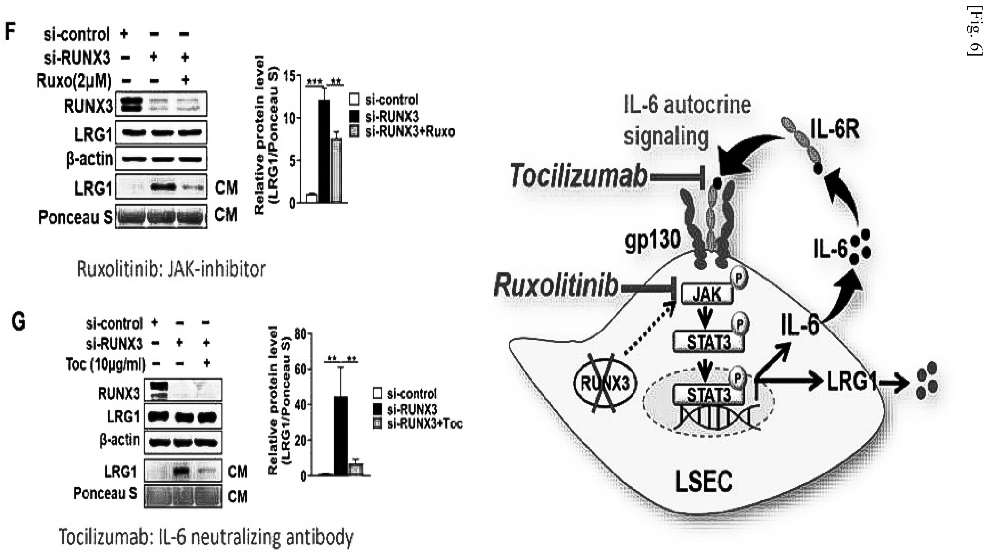

As a result, according to FIGS. 5 and 6, it was found that the expression level of Lrgl increased when Runx3 was deficient, whereas that of Lrgl decreased when Runx3 was deficient and treated together with JAK inhibitor Ruxolitinib or IL-6 neutralizing antibody Tocilizumab. This revealed that Lrgl is secreted through the IL-6/JAK/STAT3 pathway.

### <Example 4> Identification of an increase in expression levels of α-SMA and collagen I by Lrg1

Since hepatic stellate cells (hereinafter referred to as HSCs) are the main cells involved in liver fibrosis, investigation was conducted on whether Lrg-1 is involved in liver fibrosis using LX-2 cells, a hepatic stellate cell line. After the Lrg-1 recombinant protein was treated to LX-2 cells, the expression of α-SMA and collagen I was detected by Western blot.

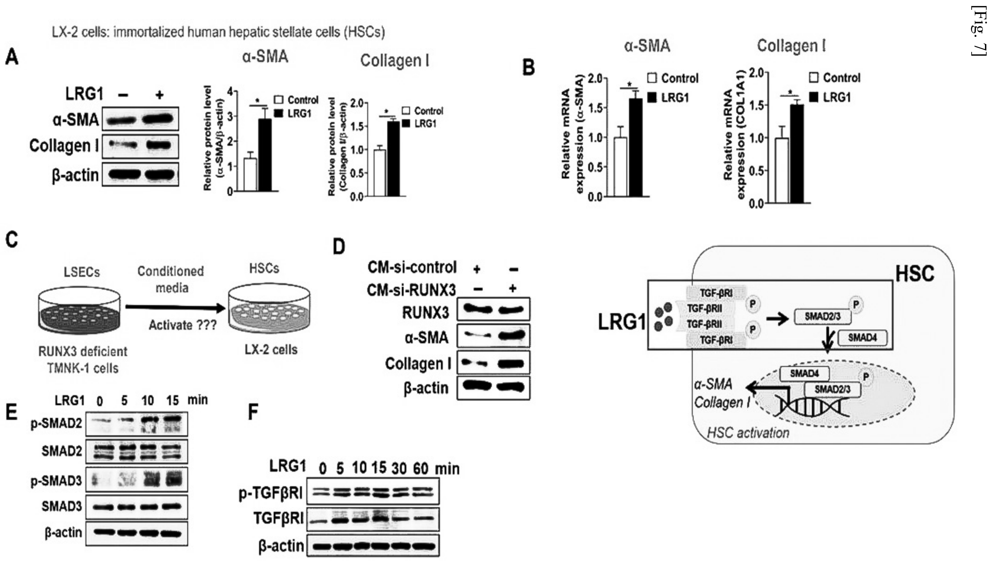

As a result, according to FIG. 7, it was found that the expression of α-SMA and collagen I increased when Lrgl was treated in HSC. This suggests that Lrgl worsens liver fibrosis.

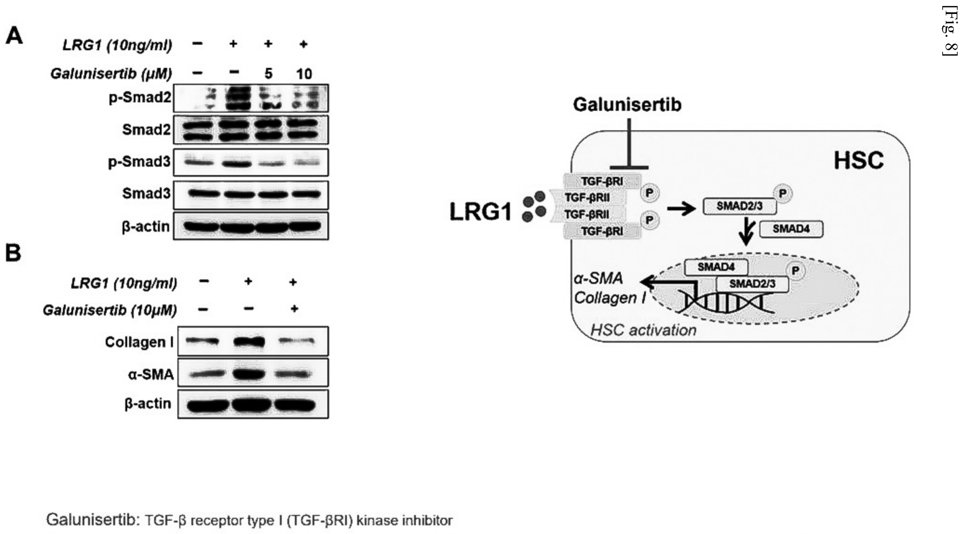

In addition, it was determined that the expression of p-SMAD2 and p-SMAD3 increased after Lrgl treatment to HSC, indicating that Lrgl passes through the p-SMAD2/p-SMAD3 pathway, and according to FIG. 8, when Galunisertib, a TGF-β receptor type 1 inhibitor, was treated, all of the α-SMA, collagen I, p-SMAD2, and p-SMAD3 increased by Lrgl treatment were decreased, indicating that Lrgl utilizes the TGF-βR/p-SMAD2/p-SMAD3 pathway.

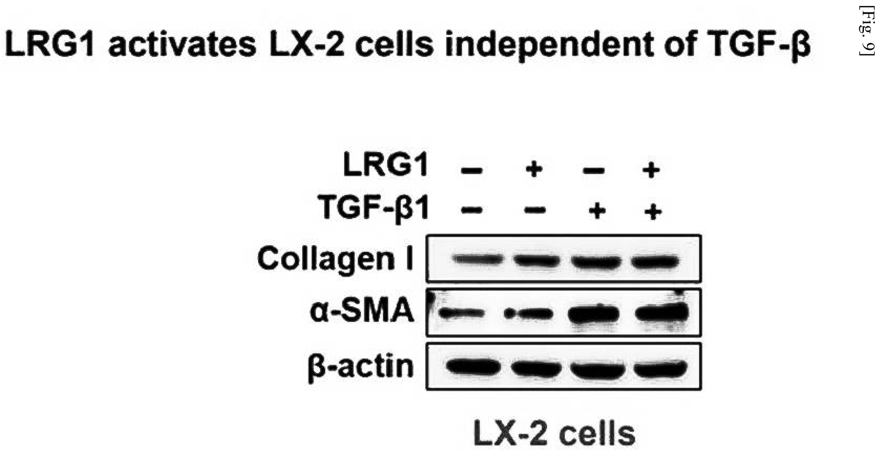

Furthermore, according to FIG. 9, when Lrgl and TGF-β were treated alone, respectively, the expression of α-SMA and collagen I increased, and when Lrgl and TGF-β were co-treated, the expression of α-SMA and collagen I also increased, indicating that Lrgl increases α-SMA and collagen I independently of TGF-β.

### <Example 5> Determination of Lrg1 as a therapeutic target for liver fibrosis

To determine that Lrgl is a therapeutic target for liver fibrosis, IHC was performed on liver tissues isolated from an animal model.

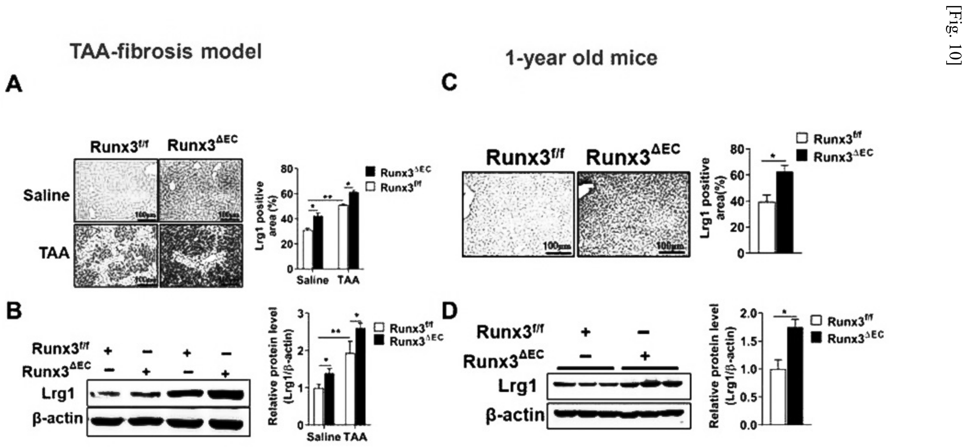

As a result, according to FIG. 10, the thioacetamide (TAA)-induced liver fibrosis model mice showed an increased expression level of Lrgl compared to the control group injected with saline, and it was also found that the expression level of Lrgl increased in the case of Runx3^{ΔEC} compared to Runx3^{f/f}. Furthermore, in the case of 1-year-old mice showing liver fibrosis symptoms, it was found that the expression amount of Lrgl increased in the case of Runx3^{ΔEC} compared to Runx3^{f/f}. This revealed that Lrgl expression increases in cases of liver fibrosis, suggesting that it may be a therapeutic target for liver fibrosis.

The foregoing description herein is for illustrative purposes only, and it will be appreciated by those skilled in the art to which the present disclosure pertains that the present disclosure may be easily modified into other specific forms without altering the technical idea or essential features of the present disclosure. Therefore, it should be understood that the examples set forth above are exemplary in all respects and not limiting.

The scope of the present disclosure is indicated by the claims described below, and all changes or modifications derived from the meaning and scope of the claims and their equivalent concepts should be construed as being included in the scope of the present disclosure.

## Claims

1. A biomarker composition for diagnosing a liver disease, comprising a leucine-rich alpha-2-glycoprotein 1 (Lrgl) protein or a gene encoding the same.

2. The biomarker composition of claim 1, wherein the liver disease is any one selected from the group consisting of liver fibrosis, liver cancer, hepatitis, hepatotoxicity, alcoholic fatty liver disease, and non-alcoholic fatty liver disease.

3. A composition for diagnosing a liver disease, containing an agent capable of measuring an expression or activity level of an Lrg1 protein, or an expression level of a gene encoding the protein as an active ingredient.

4. The composition of claim 3, wherein the agent capable of measuring the expression level of the Lrgl is a primer or probe that specifically binds to the Lrgl gene, or an antibody, peptide, aptamer, or compound that specifically binds to the Lrgl protein.

5. A kit for diagnosing a liver disease, comprising the composition according to claim 3 or 4.

6. A method of providing information necessary for diagnosing a liver disease, comprising:
(1) measuring an mRNA expression level of an Lrgl gene or an expression level of an Lrg1 protein from a sample isolated from a patient with a liver disease;
(2) comparing the mRNA expression level of the Lrgl gene or the expression level of the Lrgl protein with that of a control sample; and
(3) determining as a liver disease if the mRNA expression level of the Lrgl gene or the expression level of the Lrgl protein is higher than that of the control sample.

7. A pharmaceutical composition for preventing or treating a liver disease, comprising an Lrgl expression or activity inhibitor as an active ingredient.

8. The pharmaceutical composition of claim 7, wherein the Lrgl expression inhibitor is any one selected from the group consisting of an antisense nucleotide, a small interfering RNA (siRNA), and a short hairpin RNA (shRNA) that complementarily bind to mRNA of an Lrgl gene.

9. The pharmaceutical composition of claim 7, wherein the Lrgl activity inhibitor is any one selected from the group consisting of small molecule compounds, peptides, peptide mimetics, aptamers, antibodies, and natural products that specifically bind to an Lrgl protein.

10. A method of screening an agent for treating liver disease, the method comprising:
(1) contacting a test substance with liver disease cells;
(2) measuring an expression or activity level of an Lrgl protein in the liver disease cells that have come into contact with the test substance; and
(3) selecting a test substance having a reduced expression or activity level of the Lrgl protein compared to that of a control sample.

11. The method of claim 10, wherein the Lrgl expression level is measured by any one or more selected from the group consisting of reverse transcription-polymerase chain reaction (RT-PCR), enzyme-linked immunosorbent assay (ELISA), radioimmunoassay, immunohistochemistry, microarray, western blotting, and flow cytometry (FACS).
